# FASCICULE DE BREVET EUROPEEN

(11) **EP 1 142 566 B1**
(45) Date de publication et mention de la délivrance du brevet: **01.10.2003**
(21) Numéro de dépôt: 01107223.8
(22) Date de dépôt: 23.03.2001
(51) Int. Cl.: A61K 9/08, A61K 38/13, A61K 47/26, A61P 27/02

(54) **Formulations ophtalmiques à base de ciclosporine, d'acide hyaluronique et du polysorbate**
Cyklosporin, Hyaluronsäure und Polysorbate enthaltenes Augenarzneimittel
Compositions for ophtalmic use containing cyclosporin, hyaluronic acid and polysorbate

(30) Priorité: 07.04.2000 CH 6942000
(43) Date de publication de la demande: 10.10.2001
(73) Titulaire: LABORATOIRE MEDIDOM S.A., 1206 Geneva (CH)
(72) Inventeur: Di Napoli, Guido, 1245 Collonge-Bellerive (CH)
(74) Mandataire: Reuteler, Raymond Werner

(56) Documents cités:
- WO-A-89/01772
- WO-A-93/23010
- WO-A-95/31211
- DATABASE WPI Section Ch, Week 199315 Derwent Publications Ltd., London, GB; Class A96, AN 1993-121283 XP002172972 & JP 05 058906 A (SANKYO CO LTD), 9 mars 1993 (1993-03-09)
- GOWLAND, G. ET AL: "Marked enhanced efficacy of cyclosporin when combined with hyaluronic acid: Evidence from two T cell-mediated models" CLIN. DRUG INVEST. (1996), 11(4) 245-250, XP000613356
- GOWLAND G.: "Fourfold increase in efficiency of cyclosporin when combined with hyaluronan" INTERNATIONAL JOURNAL OF IMMUNOTHERAPY, (1998), 14(1), 1-7, XP001008242
- CONDON P I ET AL: "Double blind, randomised, placebo controlled, crossover, multicentre study to determine the efficacy of a 0.1% (w/v) sodium hyaluronate solution (Fermavisc) in the treatment of dry eye syndrome." BRITISH JOURNAL OF OPHTHALMOLOGY, (1999 OCT) 83 (10) 1121-4., XP001008246

## Description

Cette invention concerne une formulation ophtalmique topique comprenant une ciclosporine.

Les ciclosporines représentent une classe d'oligopeptides cycliques non-polaires ayant de nombreuses propriétés pharmacologiques.

Elles sont notamment connues pour leurs activités immunosuppressive et anti-inflammatoire et ont également été décrites comme efficaces pour augmenter ou rétablir la sécrétion des larmes par la glande lacrymale chez des patients souffrant de kérato-conjonctivite sèche à médiation immune.

Les ciclosporines d'origine naturelle, comprenant majoritairement la ciclosporine A et minoritairement les ciclosporines B à I, peuvent être obtenues à partir du champignon Trichoderma polysporum.

Les ciclosporines, ainsi qu'un grand nombre de leurs analogues et isomères, ont également pu être obtenus par synthèse.

Parmi les ciclosporines, la plus largement étudiée et utilisée dans le domaine pharmaceutique est la ciclosporine A.

L'activité d'une ciclosporine, et notamment de la ciclosporine A, pour augmenter ou rétablir la sécrétion des larmes par la glande lacrymale, pourrait être améliorée en améliorant l'absorption de la ciclosporine dans la glande lacrymale.

En raison de la très faible solubilité des ciclosporines dans l'eau (20-30 µg/ml pour la ciclosporine A), il a été très difficile de préparer une composition ophtalmique contenant une ciclosporine dissoute dans un milieu aqueux.

C'est pourquoi, les ciclosporines, connues comme étant lipophiles, ont principalement été utilisées dans des formulations à base d'huile.

**US-A-4.839.342** décrit une composition ophtalmique topique contenant une ciclosporine, en particulier la ciclosporine A, et un excipient, pour augmenter la production de larmes chez des patients souffrant d'un manque de larmes dans les yeux en raison d'un dysfonctionnement des glandes lacrymales. Les excipients décrits spécifiquement sont l'huile d'olive, l'huile d'arachide, l'huile de ricin, l'huile de ricin polyoxyéthylénée, les huiles minérales, les vaselines, le diméthylsulfoxyde; un alcool, les liposomes, les huiles de silicone ou les mélanges de ceux-ci.

**FR-A-2.638.089** décrit une composition ophtalmique topique contenant une ciclosporine comme substance active, et en tant que véhicule, une huile végétale comme l'huile d'olive, l'huile d'arachide, l'huile de ricin, l'huile de sésame et l'huile de maïs, et une vaseline, pour traiter les maladies et conditions immunologiques ou inflammatoires affectant l'oeil, et notamment la kérato-conjonctivite sèche KCS ou les yeux secs.

Toutefois, les formulations ophtalmiques topiques à base d'huile présentent des désavantages comme une sensation désagréable dans les yeux ou conduisent à une vision trouble. De plus, les huiles peuvent renforcer les symptômes des yeux secs.

En outre les formulations ophtalmiques topiques à base d'huile contenant une ciclosporine sont physiquement instables en raison du fait que les ciclosporines ont tendance à subir des changements de conformation et à précipiter.

De plus, ces formulations présentent une faible biodisponibilité et une tolérabilité oculaire peu élevée se manifestant par une irritation des yeux.

Dans le but de minimiser certains désavantages ci-dessus comme l'inconfort d'utilisation, et d'améliorer la biodisponibilité et la tolérabilité de la formulation, il a été proposé dans **WO 95/31211** de diminuer la quantité d'huile et de disperser la phase huileuse dans de l'eau pour former une émulsion, ce qui a conduit à une formulation ophtalmique topique sous la forme d'une émulsion à base d'eau et d'huile comprenant une ciclosporine en mélange avec un triglycéride contenant des acides gras à longue chaîne comme de l'huile de ricin et du polysorbate 80. Cette formulation contient en outre un émulsionnant, par exemple du Pemulen®.

Dans **JP-A-05 058906**, une composition ophtalmique aqueuse (gouttes oculaires),contenant de la ciclosporine A et une substance tensioactive, choisi parmi le polysorbate 80, des esters d'acides gras polyoxyéthylénés et des mélanges de ceux-ci, a été decrite.

Également dans le but de supprimer les problèmes de précipitation de la ciclosporine tout en améliorant la biodisponibilité et la tolérabilité de la formulation, il a été encore été proposé dans **US-A-5.951.971** (WO 93/23010) une formulation ophtalmique aqueuse, exempte d'huile, comprenant une ciclosporine à une concentration de 0,01 à 0,075% p/v, de l'eau, et un tensioactif dans une quantité de 0,1 à 3 % p/v destiné à améliorer la solubilité de la ciclosporine dans l'eau choisi parmi des esters d'acides gras polyoxyéthylénés, des alkylphényléthers polyoxyéthylénés, des alkyléthers polyoxyéthylénés et des mélanges de ceux-ci. Selon **US-A-5.951.971,** le polysorbate 80, également dénommé Tween 80, ne s'est pas révélé approprié comme tensioactif en raison du fait qu'il ne possède pas une activité suffisante pour solubiliser une ciclosporine dans l'eau aux concentrations désirées.

Le but de la présente invention est de supprimer les désavantages ci-dessus, notamment les problèmes de stabilité physique, et surtout d'améliorer la biodisponibilité de la formulation dans la conjonctive, la cornée et la glande lacrymale, et la tolérabilité oculaire de la formulation, en fournissant une formulation ophtalmique topique à base d'eau contenant une ciclosporine.

Ce but a été atteint après que les inventeurs aient trouvé que la présence d'acide hyaluronique et de polysorbate 80 dans une formulation ophtalmique aqueuse contenant de la ciclosporine permettait, de manière surprenante, de solubiliser la ciclosporine tout en améliorant la biodisponibilité de la formulation dans la conjonctive, la cornée et la glande lacrymale et la tolérabilité oculaire de la formulation lorsque cette formulation est administrée par voie topique dans les yeux.

L'acide hyaluronique est un mucopolysaccharide d'origine biologique, qui est largement répandu dans la nature. Il est notamment présent dans divers tissus animaux comme les cordons ombilicaux, le liquide synovial, l'humeur vitrée, les crêtes de coq et divers tissus conjonctifs comme la peau et le cartilage.

Chimiquement, l'acide hyaluronique est un membre des glycosaminoglycanes et il est constitué par des motifs alternés et répétés d'acide D-glucuronique et de N-acetyl-D-glucosamine, pour former une chaîne linéaire ayant un poids moléculaire allant jusqu'à 13 x 10⁶ Daltons.

L'utilisation pharmaceutique de l'acide hyaluronique ou d'un sel de celui-ci, notamment le hyaluronate de sodium, est largement décrite dans la littérature.

Puisque l'acide hyaluronique ou un sel de celui-ci est une substance non-immunogène et a des propriétés hydrophile et viscoélastique, il est utilisé depuis plusieurs années comme agent de remplacement du fluide vitreux de l'oeil ou comme milieu de soutien en chirurgie ophtalmique comme décrit par exemple dans **US-A-4.141.973.**

D'autres applications de l'acide hyaluronique dans le domaine de l'ophtalmologie ont également été décrites.

Ainsi, **EP-A-0.698.388** décrit une composition ophtalmique aqueuse comprenant un sel d'acide hyaluronique à une concentration de 0,05 à 2 % comme agent augmentant la viscosité pour utilisation en tant que larmes artificielles.

**WO-A-89/01772** décrit une composition ophtalmique topique à base d'huile contenant une ciclosporine pour augmenter ou rétablir la sécrétion de larmes par la glande lacrymale. L'acide hyaluronique est cité dans une liste de produits pouvant être incorporés dans la formulation en tant qu'additifs ou agents actifs additionnels.

Selon un premier aspect, la présente Invention a pour objet une formulation ophtalmique topique à base d'eau comprenant une ciclosporine, de l'acide hyaluronique ou un sel de celui-ci et du polysorbate 80.

Selon un second aspect, la présente invention a pour objet l'utilisation d'une ciclosporine en association avec de l'acide hyaluronique ou un sel de celui-ci et du polysorbate 80 pour la préparation d'une formulation à base d'eau destinée à une utilisation ophtalmique topique.

La présente invention fournit ainsi une formulation ophtalmique sous forme d'une solution aqueuse dans laquelle la ciclosporine est solubilisée sous forme micellaire, qui est stable et qui a une bonne biodisponbilité dans la conjonctive, la cornée, la glande lacrymale et l'humeur aqueuse et une tolérabilité oculaire considérablement améliorées par rapport à une formulation où la ciclosporine est solubilisée dans une émulsion eau-huile.

D'autres avantages de la présente invention apparaîtront dans la description détaillée de l'invention qui suit.

Dans la présente demande, le terme "ciclosporine" doit être compris comme incluant n'importe quel membre individuel de la classe des ciclosporines et les mélanges de ceux-ci, à moins qu'une ciclosporine particulière soit spécifiée.

Il doit également être noté que dans la présente demande, le terme "acide hyaluronique" signifie indifféremment l'acide hyaluronique sous sa forme acide ou sous la forme d'un de ses sels.

Conformément à la présente invention, la formulation de la présente invention contient une ciclosporine, de l'acide hyaluronique ou un sel de celui-ci, et du polysorbate 80.

La formulation selon la présente invention comprend de préférence 0,02 à 2 % en poids de ciclosporine, 0,01 à 2 % en poids d'acide hyaluronique ou d'un sel de celui-ci, et 0,5 à 40 % en poids de polysorbate 80, sur la base du poids total de la formulation.

Les ciclosporines qui peuvent être contenues dans la formulation de la présente invention peuvent être soit d'origine naturelle, soit d'origine synthétique.

Selon une forme d'exécution préférée, la ciclosporine contenue dans la formulation est une ciclosporine A.

Une ciclosporine A qui peut être utilisée pour préparer la formulation de la présente invention est par exemple une ciclosporine A commerciale provenant de SIGMA en Suisse.

L'acide hyaluronique contenu dans la formulation peut être soit sous sa forme acide soit sous la forme d'un de ses sels comme un hyaluronate de métal alcalin ou alcalino-terreux, par exemple le hyaluronate de sodium, le hyaluronate de potassium, le hyaluronate de magnésium, le hyaluronate de calcium ou autres.

L'acide hyaluronique ou le sel de celui-ci aura de préférence un poids moléculaire moyen en poids qui n'est pas inférieur à 1.000.000 Daltons, et plus préférentiellement un poids moléculaire moyen en poids dans la gamme de 1.300.000 à 3.000.000 Daltons. De préférence, le poids moléculaire est d'environ 1.700.000 Daltons.

De préférence, l'acide hyaluronique sera sous la forme de hyaluronate de sodium.

Le polysorbate 80, dénommé également Tween 80, est un monooléate de sorbitan polyoxyéthyléné connu pour être utilisé comme tensioactif.

Un polysorbate 80 qui peut être utilisé pour préparer la formulation de la présente invention est par exemple un polysorbate 80 commercial provenant par exemple de SIGMA.

Dans une forme d'exécution particulièrement préférée, la composition comprend 0,2 % en poids de ciclosporine A, 0,1 % en poids d'acide hyaluronique et 5 % en poids de polysorbate 80, sur la base du poids total de la formulation.

La formulation de la présente invention peut contenir entre outre des additifs comme le sorbitol qui est utilisé en tant qu'agent iso-osmotique. L'avantage du sorbitol est qu'il a un volume hydrodynamique plus grand que NaCI, par exemple. D'autres additifs possibles sont le mannitol, les polyalcools et les chlorures de sodium et de potassium.

Afin que la formulation de la présente invention soit physiologiquement acceptable, celle-ci devrait de préférence avoir un pH se situant dans une gamme de 6,5 à 7,5 et une osmolalité se situant dans une gamme de 290 à 310 mOsm/l, mais de préférence 300 mOsm/l.

La formulation de la présente invention peut être conditionnée sous forme de monodoses:

La formulation topique de la présente invention est administrée sous forme de gouttes dans les yeux et elle est utile pour augmenter ou rétablir la sécrétion des larmes par la glande lacrymale et également pour stimuler ou rétablir l'activité de la glande lacrymale, notamment chez des patients souffrant de kérato-conjonctivite sèche, du syndrome des yeux secs, du syndrome de Sjögren, de kératoconjonctivite vernale chronique, et comme prophylaxie post-opératoire de chirurgie kératoplastique.

Les exemples suivants sont destinés à illustrer la présente invention et ses avantages. Ils ne doivent en aucun cas être considérés comme limitant l'étendue de la présente invention.

### EXEMPLES

Des exemples de formulations conformes à la présente invention sont présentés dans le Tableau 1 ci-dessous.

La formulation Comp. 1 est une formulation comparative ne comprenant pas de ciclosporine.

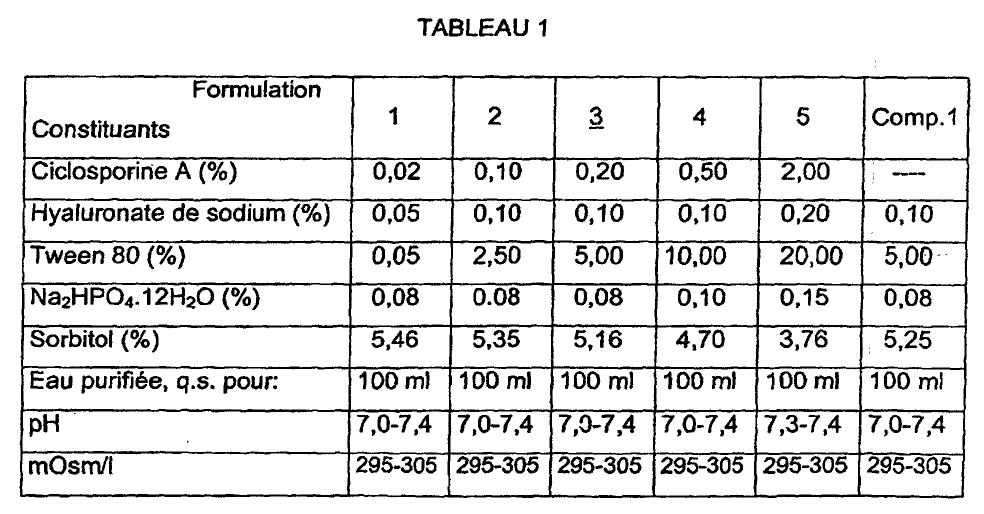

Les formulations 1 à 5 et Comp. 1 ont été soumises à des tests pour évaluer leur tolérabilité oculaire et les formulations 1 à 5 ont été soumises à un test de stabilité.

### Tolérabilité oculaire des formulations 1-5 et Comp.1

L'évaluation de la tolérabilité locale des formulations 1 à 5 et Comp.1 a été effectuée sur des lapins albinos de Nouvelle-Zélande (6 pour chaque groupe, dont 3 mâles et 3 femelles) sur lesquels on a procédé à 12 instillations de 0,1 ml chacune de formulation à tester à des intervalles de 30 minutes dans l'arcade conjonctivale droite.

L'état des tissus oculaires a été évalué conformément au test de Draize (Gad S.C. and Chengelis C.P., "Ocular Irritation Test" dans Acute Toxicology Testing. The Telford Press., Caldewell, New Jersey, USA, pp 51-80). Le test a été effectué 30 minutes après la dernière instillation. Les relevés ont été effectués par deux observateurs dans l'ignorance totale du traitement avec attribution de points arbitraires pour l'état de la conjonctive (palpébrale et bulbaire), de la cornée et de l'iris.

L'oeil traité a également été soumis au test de la fluorescéine, selon la procédure suivante. Quarante minutes après la dernière instillation de la formulation à tester, et après avoir terminé le test de Draize, on a instillé dans l'oeil à traiter une solution contenant 2 % de fluorescéine dans du sérum physiologique, et l'excès de fluorescéine a été éliminé en lavant l'oeil avec du sérum physiologique stérile. Les tissus oculaires ont ensuite été observés attentivement pour évaluer la quantité de fluorescéine qui a été absorbée. Cette évaluation faite au moyen d'une lampe à fissure a permis de démontrer la bonne tolérabilité de toutes les formulations, à savoir les formulations 1 à 5 contenant la ciclosporine A la formulation Comp.1 ne contenant pas de ciclosporine A.

Au test de Draize, la cornée et l'iris ont toujours présenté un aspect normal. La conjonctive s'est révélée normale avec absence d'oedème ou de sécrétion, excepté que dans chacun des trois groupes de lapins traité avec les formulations 3, 4 et 5 contenant respectivement 0,20 %, 0,5 % et 2 % de ciclosporine A, 2 des 6 yeux traités présentaient une hyperémie des vaisseaux dans la zone centrale, qui correspond selon le test d'évaluation à une tolérabilité encore meilleure.

Au test de la fluorescéine, il n'y avait aucune différence entre les diverses formulations; aucun des yeux traités n'a absorbé la fluorescéine.

### Stabilité des formulations 1 à 5 de la présente invention

Toutes les formulations se sont révélées stables à température ambiante, et dans aucune des formulations 1 à 5, il ne s'est produit une précipitation de la ciclosporine A dans les 12 mois qui ont suivi la préparation de la formulation.

La Formulation 3 de la présente invention a ensuite été comparée avec une émulsion de type huile-eau pour ce qui concerne la biodisponibilité de la ciclosporine A dans les tissus oculaires et la tolérabilité oculaire.

### Comparaison entre la Formulation 3 de la présente invention et une émulsion de type huile-eau.

La Formulation 3 de la présente invention a été comparée avec une émulsion huile-eau dénommée CYCLOIL, une formulation selon la demande de brevet **WO-A-95/31211**, pour ce qui concerne la tolérabilité oculaire et la biodisponibilité de la ciclosporine A dans les tissus oculaires.

La composition de ces deux formulations est résumée dans le Tableau 2 suivant.

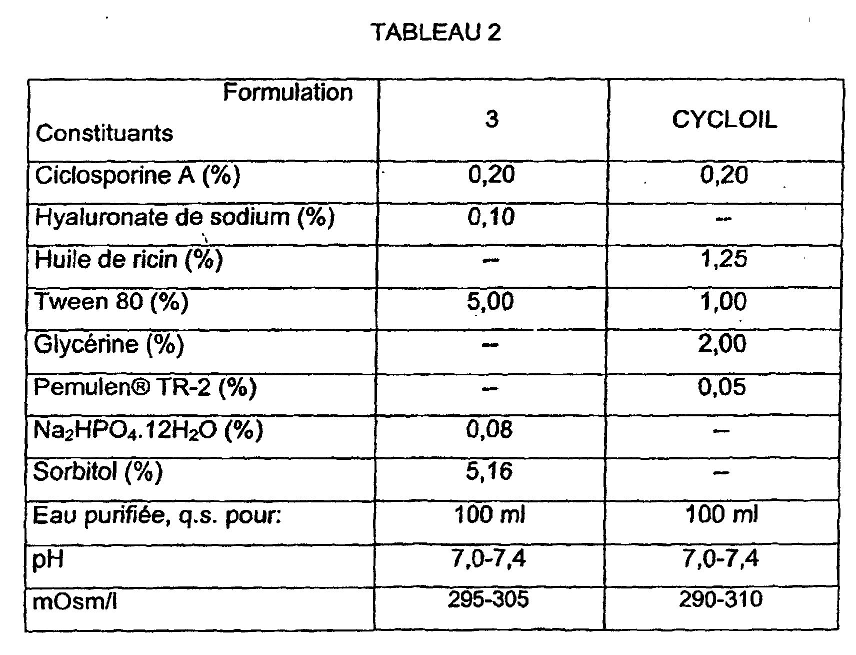

### Biodisponibilité oculaire

Dans cette expérience, il a été déterminé les concentrations de la ciclosporine A dans la conjonctive, la cornée, l'humeur aqueuse et la glande lacrymale après administration topique des deux formulations, à savoir la Formulation 3 de la présente invention et le CYCLOIL.

Les tests ont été réalisés sur des lapins mâles albinos de Nouvelle-Zélande. Les lapins ont été divisés en deux groupes de 15 lapins et ont été traités dans les deux yeux avec 50 µl par oeil, avec les deux formulations à tester. Des prélèvements de conjonctive, de cornée, d'humeur aqueuse et de glande lacrymale ont été effectués à la 1^{ère}, 3^{ème}, 6^{ème}, 12^{ème} et 24^{ème} heure après l'instillation, sur trois lapins à chaque fois pour les deux groupes après avoir sacrifié les animaux. Les yeux ont été énucléés, lavés avec une solution physiologique avant d'être soumis à un prélèvement de l'humeur aqueuse (~ 400 µl), de la cornée (~ 120 mg), de la conjonctive (~ 120 mg) et de la glande lacrymale (~ 800 mg).

La détermination quantitative de la ciclosporine A a été réalisée par HPLC en phase inverse avec élution isocratique et détection par spectroscopie UV. Le chromatographe était de marque Varian et les conditions de la chromatographie était les suivantes :
- colonne : C 18,60 x 4,6mm, 3µm (Alltech)
- phase mobile : Acétonitrile/isopropanol/H₂O (66/2/32)
- vitesse du flux : 0,7 ml/min
- température de la colonne : 72°C
- révélation : UV 205 nm (0,1-0,002 AUFS)
- volume d'injection : 25 - 50 µl
- temps de rétention : 9,1 min

Les échantillons soumis à la chromatographie ont été préparés comme suit :

### Humeur aqueuse

A 300 µl d'humeur aqueuse, il a été ajouté 150 µl d'acétonitrile et la solution ainsi obtenue a été passée au vortex pendant environ 1 minute puis ensuite centrifugée pendant 3 minutes à 3000 g. Le surnageant a été transféré dans un flacon, traité avec 15 mg de ZnSO₄ et 15 mg de CdSO₄ et passé au vortex pendant 1 minute et centrifugé pendant 2 minutes à 2000 g. La phase organique a été filtrée sur 0,45 µm et 50-75 µl ont été injectés dans la colonne.

### Cornée, conjonctive et glande lacrymale

Les tissus ont été pesés exactement, homogénéisés à froid avec du méthanol (~ 1,0 ml), centrifugés à 3000 g pendant 15 minutes, le surnageant a été repris avec du méthanol (~1 ml) et séché sous vide à environ 40°C, le résidu a été repris avec de l'acétonitrile (150 µl) traité avec du (NH₄)₂SO₄ anhydre, passé au vortex pendant 1 minute et centrifugé pendant 2 minutes à 2000 g. La phase organique a été filtrée sur 0,45 µm et 50-75 µl ont été injectés dans la colonne.

### Méthode analytique

Les concentrations de ciclosporine A dans la conjonctive, la cornée, la glande lacrymale et l'humeur aqueuse des lapins sont présentées sur les Tableaux 3 à 6.

### Concentrations de ciclosporine A dans la conjonctive

Le Tableau 3 suivant montre les concentrations (ng/g) de cyclosporine A dans la conjonctive des lapins traités avec la Formulation 3 de la présente invention et avec le CYCLOIL après 1, 3, 6, 12 et 24 heures à partir de l'instillation de 50 µl dans le sac conjonctival des deux yeux.

**TABLEAU 3**

| | Formulation 3 (invention) | CYCLOIL (comparatif) |
|---|---|---|
| concentration 1 heure après l'instillation (ng/g) | 1170 ± 170 | 820 ± 155 |
| concentration 3 heures après l'instillation (ng/g) | 900 ± 215 | 713 ± 187 |
| concentration 6 heures après l'instillation (ng/g) | 616 ± 102 | 370 ± 78 |
| concentration 12 heures après l'instillation (ng/g) | 502 ± 95 | 250 ± 70 |
| concentration 24 heures après l'instillation (ng/g) | 198 ± 40 | 75 ± 25 |
| AUC₀₋₂₄ (ng·g⁻¹·h⁻¹) | 12483 ± 234 | 7378 ± 1891 |
| Cₘₐₓ(ng/g) | 1170 ±170 | 820 ±155 |
| Tₘₐₓ (heures) | 1 | 1 |

Comme on peut le voir dans le Tableau 3 ci-dessus, la Formulation 3 de la présente invention garanti une meilleure biodisponibilité de la ciclosporine au niveau de la conjonctive à tous les moments auxquels les prélèvements ont été effectués par rapport à la formulation CYCLOIL.

La concentration maximum se trouve à la première heure, même si l'on suppose que dans la conjonctive les concentrations les plus élevées de ciclosporine en absolu se trouveraient juste après l'instillation.

La Formulation 3 de la présente invention à base d'eau garanti pour tous les prélèvements des concentrations en ciclosporine A toujours plus élevées par rapport à l'émulsion huile-eau CYCLOIL.

Les AUC (Area Under the Curve) sont respectivement de 12483 ± 234 ng·g⁻¹·h⁻¹ pour la Formulation 3 de la présente invention et de 7378 ± 1891 ng·g⁻¹·h⁻¹ pour l'émulsion huile-eau CYCLOIL.

La même chose se produit au niveau de la cornée.

### Concentrations de ciclosporine A dans la cornée

Le Tableau 4 montre les concentrations (ng/g) de cyclosporine A dans la cornée des lapins traités avec la Formulation 3 de la présente invention et avec le CYCLOIL à base d'huile après 1, 3, 6, 12 et 24 heure après l'instillation de 50 µl dans le sac conjonctival des deux yeux.

**TABLEAU 4**

| | Formulation 3 (invention) | CYCLOIL (comparatif) |
|---|---|---|
| concentration 1 heure après l'instillation | 2995 ± 750 | 2070 ± 1115 |
| concentration 3 heures après l'instillation | 3350 ± 920 | 1991 ± 630 |
| concentration 6 heures après l'instillation | 2520 ± 870 | 2420 ± 870 |
| concentration 12 heures après l'instillation | 2228 ± 490 | 1825 ± 690 |
| concentration 24 heures après l'instillation | 1590 ± 220 | 450 ± 190 |
| AUC₀₋₂₄ (ng·g⁻¹·h⁻¹) | 53800 ± 13070 | 38097 ± 1397 |
| Cₘₐₓ (ng/g) | 3350 ±920 | 2420 ± 870 |
| Tₘₐₓ (heures) | 3 | 6 |

Comme on peut le voir dans le Tableau 4 ci-dessus, la concentration maximum du médicament s'obtient à la troisième heure pour la Formulation 3 de la présente invention (3350 ±920 ng/g) et à la 6^{ème} heure pour le CYCLOIL (2420 ± 870 ng/g).

La Formulation 3 de la présente invention à base d'eau garantit pour tous les prélèvements des concentrations en ciclosporine A toujours plus élevées par rapport à l'émulsion huile-eau CYCLOIL.

### Concentrations de ciclosporine A dans la glande lacrymale

Le Tableau 5 montre les concentrations (ng/g) de cyclosporine A dans la glande lacrymale des lapins traités avec la Formulation 3 de la présente invention et avec le CYCLOIL à base d'huile après 1, 3, 6, 12 et 24 heures après l'instillation de 50 µl dans le sac conjonctival des deux yeux.

**TABLEAU 5**

| | Formulation 3 (invention) | CYCLOIL (comparatif) |
|---|---|---|
| concentration 1 heure après l'instillation (ng/g) | 88 ± 29 | 22 ± 10 |
| concentration 3 heures après l'instillation (ng/g) | 149 ± 45 | 42 ± 16 |
| concentration 6 heures après l'instillation (ng/g) | 135 ± 33 | 53 ± 18 |
| concentration 12 heures après l'instillation (ng/g) | 54 ± 22 | 21 ± 10 |
| concentration 24 heures après l'instillation (ng/g) | 38 ± 19 | 17 ± 10 |
| AUC₀₋₂₄ (ng·g⁻¹·h⁻¹) | 1826 ± 616 | 668 ± 286 |
| Cₘₐₓ(ng/g) | 149 ± 45 | 53 ± 18 |
| Tₘₐₓ (heures) | 3 | 6 |

La Formulation 3 de la présente invention à base d'eau présente une concentration maximale de ciclosporine A à la 3^{ème} heure (149 ± 45 ng/g) alors que le CYCLOIL présente une concentration maximale de la ciclosporine de 53 ± 16 ng/g à la 6^{ème} heure.

Comme prévu, les concentrations de ciclosporine A dans la glande lacrymale qui ont été reportées sur le Tableau 5 sont nettement plus faibles que celles obtenues dans la conjonctive et dans la cornée, mais elles sont toujours supérieures à la limite de sensibilité de la méthode (15 ng/g).

Comme on peut le voir ci-dessus, la biodisponibilité de la Formulation 3 de la présente invention est beaucoup plus élevée que celle du CYCLOIL et de plus, la ciclosporine A est encore présente 24 heures après l'instillation bien qu'à des concentrations plus faibles, mais mesurables. Le rapport AUC_{AN-023}/AUC_{cycloil}, qui représente le rapport entre les biodisponibilité, est d'environ 3.

### Concentrations de ciclosporine A dans l'humeur aqueuse

Le Tableau 6 montre les concentrations (ng/g) de ciclosporine A dans l'humeur aqueuse des lapins traités avec la Formulation 3 de la présente invention et avec le CYCLOIL à base d'huile après 1, 3, 6, 12 et 24 heure après l'instillation de 50 µl dans le sac conjonctival des deux yeux.

**TABLEAU 6**

| | Formulation 3 (invention) | CYCLOIL (comparatif) |
|---|---|---|
| concentration 1 heure après l'instillation (ng/g) | 40 ± 12 | 12 ± 13 |
| concentration 3 heures après l'instillation (ng/g) | 41 ± 14 | 18 ± 7 |
| concentration 6 heures après l'instillation (ng/g) | 31 ± 8 | 22 ± 11 |
| concentration 12 heures après l'instillation (ng/g) | 27 ± 9 | 16 ± 4 |
| concentration 24 heures après l'instillation (ng/g) | 16 ± 5 | 14 ± 2 |
| AUC₀₋₂₄ (ng.g⁻¹·h⁻¹) | 641 ± 14 | 390 ± 119 |
| Cₘₐₓ(ng/g) | 41 ± 14 | 22 ± 11 |
| Tₘₐₓ (heures) | 3 | 6 |

La concentration maximale de ciclosporine instillée en formulation aqueuse (Formulation 3 de la présente invention) s'est révélée à la 3^{ème} heure (44 ± 14 ng/ml) alors que pour l'émulsion à base d'huile CYCLOIL, le maximum s'est révélé à la 6^{ème} heure (22 ± 11 ng/ml)

Les concentrations de ciclosporine A dans l'humeur aqueuse des lapins qui on été traités par instillation de 50 µl avec les deux formulations testées se sont révélées relativement basses par rapport aux deux tissus qui constituent la superficie oculaire (cornée et conjonctive). Cependant, pour l'humeur aqueuse comme pour les autres tissus oculaires, la biodisponibilité de la ciclosporine administrée en formulation aqueuse était plus élevée que celle instillée en émulsion eau-huile.

### Evaluation de la tolérabilité

La tolérabilité de la Formulation 3 de la présente invention et du CYCLOIL a été évaluée conformément au test de Draize modifié décrit précédemment.

Deux groupes de 6 animaux, comprenant 3 mâles et 3 femelles lapins albinos de Nouvelle-Zélande, ont été traités dans l'oeil droit en réalisant 12 instillations de 0,1 ml des deux préparations à tester, pendant 6 heures à des intervalles de 30 minutes l'une de l'autre.

Le test de Draize pour évaluer la tolérabilité a été réalisé 30 minutes après la dernière instillation. Après ce test, 40 minutes après la dernière instillation, le test de la fluorescéine a été effectué. Les résultat de ces deux tests sont reportés dans les Tableaux 7 et 8.

Dans le Tableau 7 suivant, il a été reporté, conformément au test de Draize, le degré de rougeoiement de la conjonctive des lapins traités avec la Formulation 3 de la présente invention et avec le CYCLOIL par instillation dans l'oeil droit. Le rougeoiement a été évalué selon une échelle arbitraire. Le traitement a été effectué 12 fois à des intervalles de 30 minutes. L'évaluation a été faite 30 minutes après la dernière instillation.

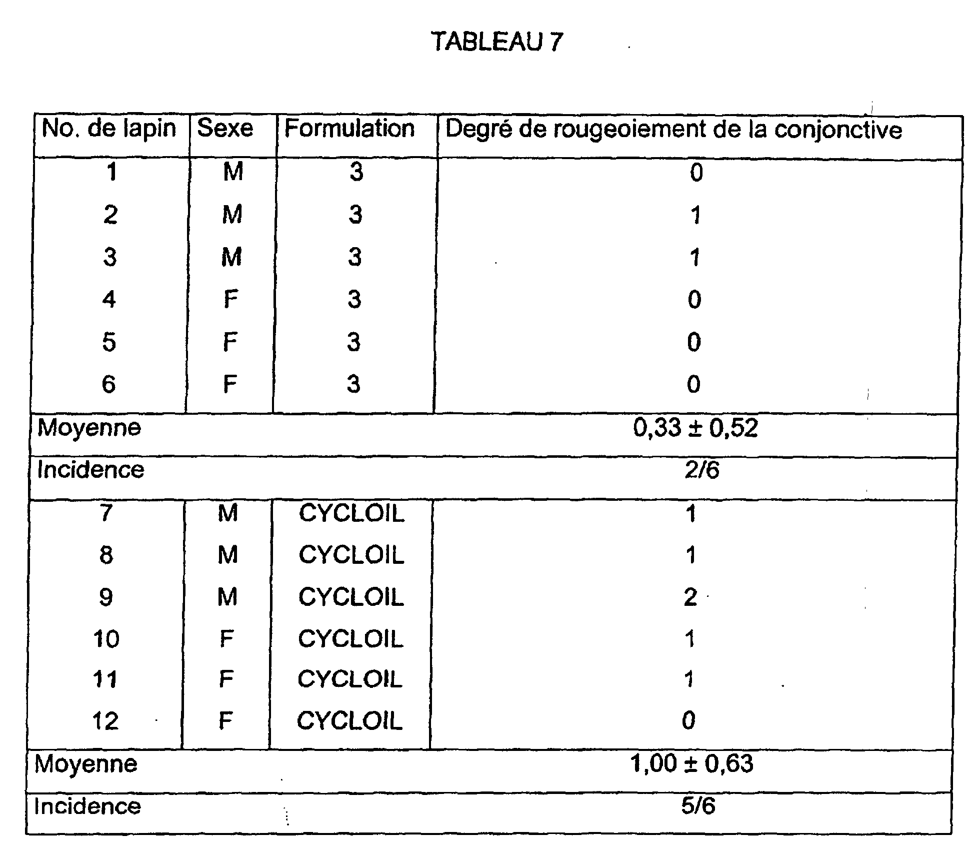

Comme on peut le voir, la Formulation 3 de la présente invention a donné seulement 2 cas sur 6 de rougeoiement, avec une certaine hyperémie dans la zone centrale, alors que le CYCLOIL à base d'huile montrait une incidence de 5 lapins sur 6. Avec le CYCLOIL, le degré de rougeoiement pour 4 lapins était faible mais 1 heure après la dernière instillation, un lapin présentait une hyperémie diffuse de couleur rouge cramoisi où les vaisseaux individuels étaient difficile à distinguer.

Aucun des lapins traités n'a présenté un oedème ou une sécrétion supérieure à la normale au niveau de la conjonctive.

Dans le Tableau 8 suivant, il a été reporté le degré d'hyperémie de l'iris des lapins traités avec la Formulation 3 de la présente invention et avec le CYCLOIL par instillation dans l'oeil droit. L'hyperémie indienne a été évalue au moyen d'une échelle arbitraire. Le traitement a été effectué 12 fois à des intervalles de 30 minutes. L'évaluation a été effectuée 30 minutes après la dernière instillation.

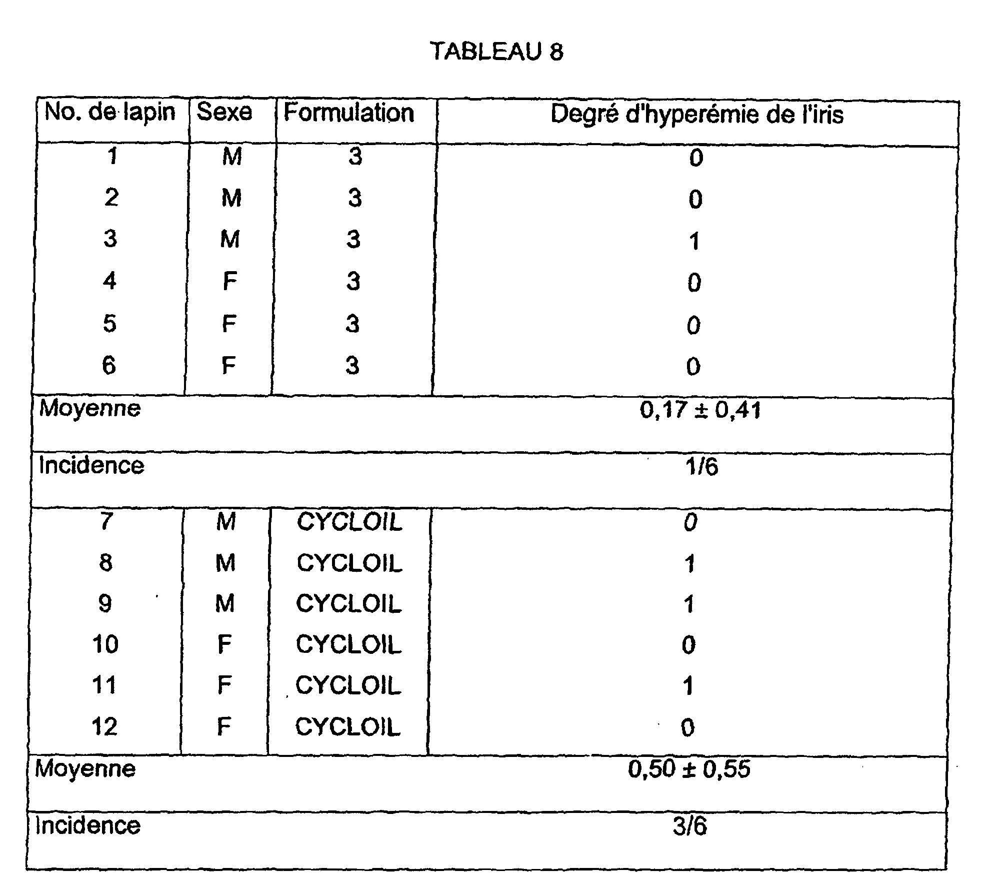

Seulement un lapin du groupe traité avec la Formulation 3 de la présente invention a présenté une hyperémie minimale à charge des vaisseaux secondaires de l'iris, et non des vaisseaux tertiaires.

L'incidence de ce type d'hyperémie, très légère, a été de 3 sur 6 dans le groupe traité avec le CYCLOIL.

Sur le Tableau 9 ci-après, il a été reporté le degré d'opacité cornéenne des lapins traités avec la Formulation 3 selon l'invention et avec le CYCLOIL instillés dans l'oeil droit. L'opacité a été évaluée selon une échelle arbitraire. Le traitement a été effectué 12 fois à des intervalles de 30 minutes. L'évaluation a été effectuée 30 minutes après la dernière instillation.

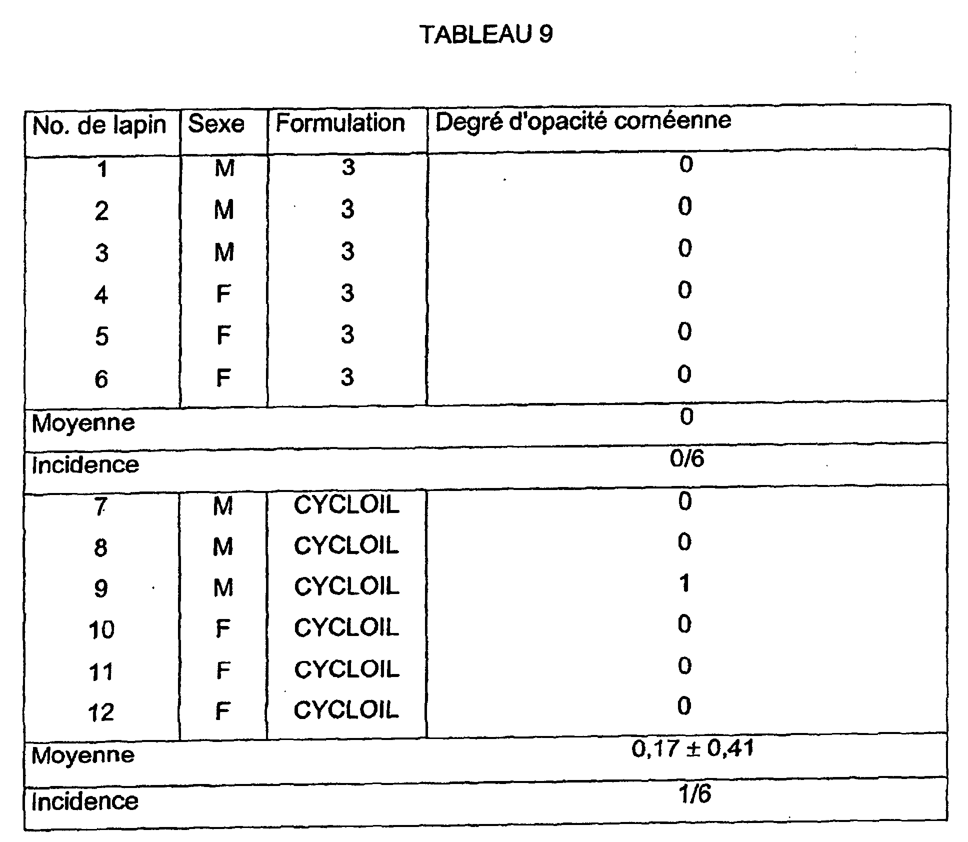

Seulement un lapin du groupe traité avec le CYCLOIL a présenté une zone d'opacité cornéenne dispersée, qui permettait cependant toujours une bonne visibilité de l'iris.

Dans le Tableau 10 ci-après, on a reporté le degré d'absorption de la fluorescéine dans l'épithélium cornéen des lapins traités avec la Formulation 3 selon l'invention et avec le CYCLOIL instillés dans l'oeil droit. L'absorption a été évaluée selon une échelle arbitraire. Le traitement a été effectué 12 fois à des intervalles de 30 minutes. L'évaluation a été effectuée 40 minutes après la dernière instillation.

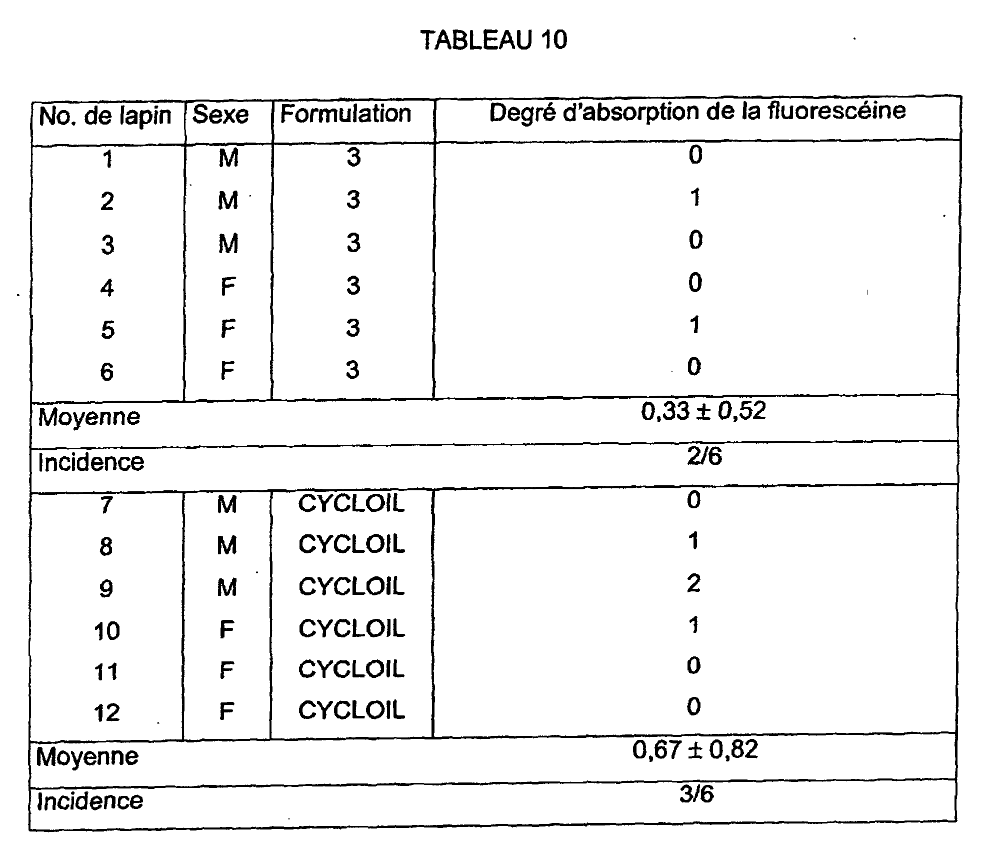

Comme on peut le voir ci-dessus, la Formulation 3 de la présente invention s'est également révélée mieux tolérée au test de la fluorescéine. En fait, 3 cornées du groupe traité avec le CYCLOIL avaient absorbé la fluorescéine et une de celle-ci, celle du lapin No. 9, a montré la présence de points fluorescents accentués, même si les structures des divers tissus se révélaient quand même distinguables, quoique qu'avec une perte de détails lorsqu'un éclairage approprié était utilisé.

Seuls 2 des six lapins traités avec la Formulation 3 de la présente invention montraient de rares petits points fluorescents avec aucune coloration visible autour du bord pupillaire externe.

Les résultats ci-dessus démontrent ainsi clairement que la formulation aqueuse selon la présente invention s'est révélée non seulement mieux tolérée mais également plus biodisponible qu'une émulsion huile-eau à base d'huile de ricin, de Tween 80 (polysorbate 80), de glycérine et de Pemulen® TR-2 (CYCLOIL).

## Revendications

1. Formulation ophtalmique topique sous forme de solution aqueuse comprenant une ciclosporine, de l'acide hyaluronique ou un sel de celui-ci, et du polysorbate 80.

2. Formulation selon la revendication 1, **caractérisée en ce qu'**elle comprend 0,02 à 2 % en poids de ciclosporine, 0,01 à 2 % en poids d'acide hyaluronique ou d'un sel de celui-ci, et 0,5 à 40 % en poids de polysorbate 80, sur la base du poids total de la formulation.

3. Formulation selon la revendication 1 ou 2, **caractérisée en ce que** la ciclosporine est une ciclosporine A.

4. Formulation selon l'une quelconque des revendications 1 à 3, **caractérisée en ce que** l'acide hyaluronique ou le sel de celui-ci a un poids moléculaire moyen en poids qui n'est pas inférieur à 1.300.000 Daltons.

5. Formulation selon la revendication 4, **caractérisée en ce que** l'acide hyaluronique ou le sel de celui-ci a un poids moléculaire moyen en poids se situant dans la gamme de 1.300.000 à 3.000.000 Daltons.

6. Formulation selon l'une quelconque des revendications 1 à 5, **caractérisée ce que** l'acide hyaluronique est sous la forme de hyaluronate de métal alcalin ou alcalino-terreux.

7. Formulation selon la revendication 6, **caractérisée en ce que** l'acide hyaluronique est sous la forme de hyaluronate de sodium.

8. Formulation selon l'une quelconque des revendications 2 à 7, **caractérisée en ce qu'**elle comprend 0,2 % en poids de ciclosporine A, 0,1 % en poids d'acide hyaluronique ou d'un sel de celui-ci et 5 % en poids de polysorbate 80, sur la base du poids total de la formulation.

9. Formulation selon l'une quelconque des revendications 1 à 8, **caractérisée en ce qu'**elle comprend en outre des additifs.

10. Formulation selon l'une quelconque des revendications 1 à 9, pour utilisation sous forme de gouttes pour les yeux pour augmenter ou rétablir la sécrétion des larmes par la glande lacrymale, pour stimuler ou rétablir l'activité de la glande lacrymale, et comme prophylaxie post-opératoire de chirurgie kératoplastique.

11. Utilisation d'une ciclosporine en association avec de l'acide hyaluronique ou un sel de celui-ci et du polysorbate 80 pour la préparation d'une formulation ophtalmique sous forme de solution aqueuse destinée à une administration topique dans les yeux pour augmenter ou rétablir la sécrétion des larmes par la glande lacrymale, pour stimuler ou rétablir l'activité de la glande lacrymale, et comme prophylaxie post-opératoire de chirurgie kératoplastique.

12. Utilisation selon la revendication 11, dans laquelle la formulation comprend 0,02 à 2 % en poids de ciclosporine, 0,01 à 2 % en poids d'acide hyaluronique ou d'un sel de celui-ci, et 0,5 à 40 % en poids de polysorbate 80, sur la base du poids total de la formulation.

13. Utilisation selon la revendication 11 ou 12, **caractérisée en ce que** la ciclosporine est une ciclosporine A.

14. Utilisation selon l'une quelconque des revendications 11 à 13, **caractérisée en ce que** l'acide hyaluronique ou le sel de celui-ci a un poids moléculaire moyen en poids qui n'est pas inférieur à 1.300.000 Daltons.

15. Utilisation selon la revendication 14, **caractérisée en ce que** l'acide hyaluronique ou le sel de celui-ci a un poids moléculaire moyen en poids se situant dans la gamme de 1.300.000 à 3.000.000 Daltons.

16. Utilisation selon l'une quelconque des revendications 11 à 15, **caractérisée ce que** l'acide hyaluronique est sous la forme de hyaluronate de métal alcalin ou alcalino-terreux.

17. Utilisation selon la revendicatio 16, **caractérisée en ce que** l'acide hyaluronique est sous la forme de hyaluronate de sodium.

18. Utilisation selon l'une quelconque des revendications 11 à 17, **caractérisée en ce que** la formulation est destinée au traitement d'une kérato-conjonctivite sèche (KCS).

19. Utilisation selon l'une quelconque des revendications 11 à 17, **caractérisée en ce que** la formulation est destinée au traitement du syndrome de Sjögren.

20. Utilisation selon l'une quelconque des revendications 11 à 17, **caractérisée en ce que** la formulation est destinée au traitement du syndrome des yeux secs.

21. Utilisation selon l'une quelconque des revendications 11 à 17, **caractérisée en ce que** la formulation est destinée au traitement de la kérato-conjonctivite vernale chronique.

22. Utilisation selon l'une quelconque des revendications 11 à 17, **caractérisée en ce que** la formulation est destinée à l'utilisation en tant que prophylaxie post-opératoire de chirurgie kératoplastique.

## Patentansprüche

1. Topische ophthalmische Rezeptur in Gestalt einer wässrigen Lösung mit einem Cyclosporin, Hyaluronsäure oder einem ihrer Salze sowie Polysorbat 80.

2. Rezeptur gemäss Anspruch 1, **dadurch gekennzeichnet, dass** sie 0,02 bis 2 Gewichtsprozent an Cyclosporin, 0,01 bis 2 Gewichtsprozent an Hyaluronsäure oder einem ihrer Salze sowie 0,5 bis 40 Gewichtsprozent an Polysorbat 80, bezogen auf das Gesamtgewicht der Rezeptur, enthält.

3. Rezeptur gemäss Anspruch 1 oder 2, **dadurch gekennzeichnet, dass** das Cyclosporin ein Cyclosporin A ist.

4. Rezeptur nach einem beliebigen der Ansprüche 1 bis 3, **dadurch gekennzeichnet, dass** die Hyaluronsäure oder ihr Salz ein Gewichtsmittel des Molekulargewichgts von nicht weniger als 1 300 000 Dalton besitzt.

5. Rezeptur gemäss Anspruch 4, **dadurch gekennzeichnet, dass** die Hyaluronsäure oder ihr Salz ein Gewichtsmittel des Molekulargewichts hat, das im Bereich von 1 300 000 bis 3 000 000 Dalton liegt.

6. Rezeptur nach einem beliebigen der Ansprüche 1 bis 5, **dadurch gekennzeichnet, dass** die Hyaluronsäure in Gestalt von Alkali- oder Erdalkalimetallhyaluronat vorliegt.

7. Rezeptur nach Anspruch 6, **dadurch gekennzeichnet, dass** die Hyaluronsäure in Gestalt von Natriumhyaluronat vorliegt.

8. Rezeptur nach einem beliebigen der Ansprüche 2 bis 7, **dadurch gekennzeichnet, dass** sie 0,2 Gewichtsprozent an Cyclosporin A, 0,1 Gewichtsprozent an Hyaluronsäure oder einem ihrer Salze und 5 Gewichtsprozent an Polysorbat, bezogen auf das Gesamtgewicht der Rezeptur enthält.

9. Rezeptur nach einem beliebigen der Ansprüche 1 bis 8, **dadurch gekennzeichnet, dass** sie ausserdem Additive umfasst.

10. Rezeptur nach einem beliebigen der Ansprüche 1 bis 9 zum Einsatz in Gestalt von Augentropfen, um die Tränensekretion durch die Tränendrüse zu erhöhen oder wiederherzustellen, die Aktivität der Tränendrüse anzuregen oder wiederherzustellen, und als postoperative Prophylaxe bei der Keratoplastik.

11. Verwendung eines Cyclosporins in Gemeinschaft mit Hyaluronsäure oder einem ihrer Salze sowie mit Polysorbat 80 für die Zubereitung einer ophthalmischen Rezeptur in Gestalt einer wässrigen Lösung, die für eine lokale Verabreichung in die Augen bestimmt ist, um die Tränensekretion durch die Tränendrüse zu erhöhen oder wiederherzustellen, die Aktivität der Tränendrüse anzuregen oder wiederherzustellen, und als postoperative Prophylaxe bei der Keratoplastik.

12. Verwendung gemäss Anspruch 11, bei der die Rezeptur 0,02 Gewichtsprozent an Cyclosporin, 0,01 bis 2 Gewichtsprozent an Hyaluronsäure oder einem ihrer Salze sowie 0,5 bis 40 Gewichtsprozent an Polysorbat 80, bezogen auf das Gesamtgewicht der Rezeptur enthält.

13. Verwendung gemäss Anspruch 11 oder 12, **dadurch gekennzeichnet, dass** das Cyclosporin ein Cyclosporin A ist.

14. Verwendung nach einem beliebigen der Ansprüche 11 bis 13, **dadurch gekennzeichnet, dass** die Hyaluronsäure oder ihr Salz ein Gewichtsmittel des Molekulargewichts von nicht weniger als 1 300 000 Dalton besitzt.

15. Verwendung gemäss Anspruch 14, **dadurch gekennzeichnet, dass** die Hyaluronsäure oder ihr Salz ein Gewichtsmittel des Molekulargewichts besitzt, das im Bereich von 1 300 000 bis 3 000 000 Dalton liegt.

16. Verwendung nach einem beliebigen der Ansprüche 11 bis 15, **dadurch gekennzeichnet, dass** die Hyaluronsäure in Gestalt von Alkali- oder Erdalkalimetallhyaluronat vorliegt.

17. Verwendung gemäss Anspruch 16, **dadurch gekennzeichnet, dass** die Hyaluronsäure in Gestalt von Natriumhyaluronat vorliegt.

18. Verwendung nach einem beliebigen der Ansprüche 11 bis 17, **dadurch gekennzeichnet, dass** die Rezeptur für die Behandlung einer Keratoconjunctivitis sicca (KCS) bestimmt ist.

19. Verwendung nach einem beliebigen der Ansprüche 11 bis 17, **dadurch gekennzeichnet, dass** die Rezeptur für die Behandlung des Sjögren-Syndroms bestimmt ist.

20. Verwendung nach einem beliebigen der Ansprüche 11 bis 17, **dadurch gekennzeichnet, dass** die Rezeptur für die Behandlung des Sicca-Syndroms bestimmt ist.

21. Verwendung nach einem beliebigen der Ansprüche 11 bis 17, **dadurch gekennzeichnet, dass** die Rezeptur für die Behandlung der chronischen Frühjahrs-Konjunktivitis bestimmt ist.

22. Verwendung nach einem beliebigen der Ansprüche 11 bis 17, **dadurch gekennzeichnet, dass** die Rezeptur für die Verwendung als postoperative Prophylaxe der Keratoplastik bestimmt ist.

## Claims

1. Topical ophthalmic formulation in the form of an aqueous solution comprising a cyclosporin, hyaluronic acid or one of its salts, and polysorbate 80.

2. Formulation according to claim 1, **characterized in that** it comprises 0.02 to 2 % by weight of cyclosporin, 0.01 to 2 % by weight of hyaluronic acid or one of its salts, and 0.5 to 40 % by weight of polysorbate 80, based on the formulation's total weight.

3. Formulation according to claim 1 or 2, **characterized in that** the cyclosporin is a cyclosporin A.

4. Formulation according to any one of claims 1 to 3, **characterized in that** the hyaluronic acid or its salt has a weight-average molecular weight not inferior to 1,300,000 daltons.

5. Formulation according to claim 4, **characterized in that** the hyaluronic acid or its salt has a weight-average molecular weight situated in the region from 1,300,000 to 3,000,000 daltons.

6. Formulation according to any one of claims 1 to 5, **characterized in that** the hyaluronic acid is present as alkali metal or alkaline-earth metal hyaluronate.

7. Formulation according to claim 6, **characterized in that** the hyaluronic acid is present as sodium hyaluronate.

8. Formulation according to any one of claims 2 to 7, **characterized in that** it comprises 0.2 % by weight of cyclosporin A, 0.1 % by weight of hyaluronic acid or one of its salts, and 5 % by weight of polysorbate 80, based on the formulation's total weight.

9. Formulation according to any one of claims 1 to 8, **characterized in that** in addition it comprises additives.

10. Formulation according to any one of claims 1 to 9, for use as eye drops for increasing or re-establishing tear secretion by the lacrimal gland, for stimulation or re-establishing activity of the lacrimal gland, and as post-operative prophylactic in keratoplasty.

11. Use of a cyclosporin in association with hyaluronic acid or one of its salts and with polysorbate 80 for the preparation of a formulation in the form of an aqueous solution intended for topical ophthalmic use for stimulation or re-establishing activity of the lacrimal gland, and as post-operative prophylactic in keratoplasty.

12. Use according to claim 11 where the formulation comprises 0.02 to 2 % by weight of cyclosporin, 0.01 to 2 % by weight of hyaluronic acid or one of its salts, and 0.5 to 40 % by weight of polysorbate 80, based on the formulation's total weight.

13. Use according to claim 11 or 12, **characterized in that** the cyclosporin is a cyclosporin A.

14. Use according to any one of claims 11 to 13, **characterized in that** the hyaluronic acid or its salt has a weight-average molecular weight not inferior to 1,300,000 daltons.

15. Use according to claim 14, **characterized in that** the hyaluronic acid or its salt has a weight-average molecular weight situated in the region from 1,300,000 to 3,000,000 daltons.

16. Use according to any one of claims 11 to 15, **characterized in that** the hyaluronic acid is present as alkali metal or alkaline-earth metal hyaluronate.

17. Use according to claim 16, **characterized in that** the hyaluronic acid is present as sodium hyaluronate.

18. Use according to any one of claims 1 to 17, **characterized in that** the formulation is intended for the treatment of a keratoconjunctivitis sicca (KCS).

19. Use according to any one of claims 1 to 17, **characterized in that** the formulation is intended for the treatment of Sjögren's syndrome.

20. Use according to any one of claims 1 to 17, **characterized in that** the formulation is intended for the treatment of dry-eye syndrome.

21. Use according to any one of claims 1 to 17, **characterized in that** the formulation is intended for the treatment of chronic vernal keratoconjunctivitis.

22. Use according to any one of claims 1 to 17, **characterized in that** the formulation is intended for use as a post-operative prophylactic in keratoplasty.
